(19)

Europäisches Patentamt
European Patent Office
Office européen des brevets

(11) **EP 4 431 968 A1**

(12) **EUROPEAN PATENT APPLICATION**

(43) Date of publication:
**18.09.2024 Bulletin 2024/38**

(21) Application number: **23162592.2**

(22) Date of filing: **17.03.2023**

(51) International Patent Classification (IPC):
**G01R 33/56** (2006.01)   **G01R 33/24** (2006.01)
**G01R 33/561** (2006.01)   **G06N 3/02** (2006.01)

(52) Cooperative Patent Classification (CPC):
**G01R 33/5608; G01R 33/246; G01R 33/5612;**
G06N 20/20

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Siemens Healthineers AG**
 **91301 Forchheim (DE)**
• **King's College London**
 **London WC2R 2LS (GB)**

(72) Inventors:
• **Dubiner, Michael**
 **London, NW11 9LA (GB)**
• **Hajnal, Joseph V.**
 **London, NW34PY (GB)**
• **Malik, Shaihan**
 **London, N20 9LL (GB)**
• **Neji, Radhouene**
 **London, N65XP (GB)**
• **Tomi-Tricot, Raphael**
 **LONDON, SW11 2JP (GB)**

(74) Representative: **Siemens Healthineers Patent Attorneys**
 **Postfach 22 16 34**
 **80506 München (DE)**

(54) **COMPUTING SYSTEM FOR PROVIDING A MAPPING OF A PHYSICAL QUANTITY ON A BIOLOGICAL TISSUE AND METHOD THEREOF**

(57)    A computing system (100) for providing a mapping of a physical quantity on a biological tissue, comprising: an input data interface (10), configured to obtain data from the physical quantity on different spatial points of the biological tissue; a computation module (20), having at least: (a) a digitization unit (220), configured to produce a digitized representation of the biological tissue in voxels and/or pixels, wherein, based on the obtained data, a subset of the voxels and/or pixels is assigned a value of the physical quantity; (b) a concatenation unit (230), configured to spatially correlate the voxels and/or pixels of the produced digitized representation of the biological tissue; and (c) a regression unit (240), configured to process the information of the spatially correlated voxels and/or pixels and generate a regression analysis of the physical quantity on the biological tissue; and an output data interface (30), configured to, based on the generated regression analysis, provide a mapping of the physical quantity on the biological tissue, wherein a value of the physical quantity is assigned to each voxel and/or pixel.

FIG 1

# Description

The present invention relates to a computing system for providing mappings of physical quantities present on biological tissues. The present invention further relates to a computer-implemented method for providing such mappings.

The invention is mostly described with respect to the determination of radiofrequency (RF) magnetic fields in the context of magnetic resonance imaging (MRI), especially in ultrahigh field MRI with parallel RF transmission, but the principles of the invention have a broader scope and apply equally well to other medical and/or clinical applications.

Herein and in the forthcoming, independent of the grammatical term usage, individuals with male, female or other gender identities are included within the term.

A wide variety of advanced medical techniques are based on reliable determinations of the physical properties on biological tissues. Examples thereof are the determination of the temperature distribution of different organs (e.g., liver, brain, kidney) during a resection, the velocity field of corporal fluids (e.g. in the heart) or the determination of magnetic fields in soft tissue, e.g. during a magnetic resonance scan.

Quite generically, a determination of physical quantities on biological tissues involves measurement techniques that require long acquisition times. Magnetic resonance imaging (MRI) provides several examples of this sort. MRI is a non-invasive medical imaging technique for the examination of soft body tissues based on the detection of the relaxation times of proton spins, which originate as a response to pulses of computer-generated radio waves. Data acquisition can take of the order of minutes, during which patients are requested to do breath holds in order to reduce the abdominal make it hard to image structures like the heart or the blood, which have rather short characteristic times.

In order to ameliorate this problem, different measurement techniques have been developed, with the aim to reduce the acquisition time at the expense of accuracy, hoping to reach an acceptable balance between the two.

In MRI, such a situation takes place in the evaluation of the radio-frequency magnetic field $B_1$. One measurement mechanism is Actual Flip angle Imaging (AFI), which is very accurate but requires a lengthy acquisition time (of the order of minutes). Measurement alternatives are provided by different variants based on Fast Low Angle Shot magnetic resonance imaging (FLASH imaging). Such alternatives require acquisition times of the order of seconds but in some cases (e.g. in MRI with ultrahigh magnetic fields), where the RF magnetic field is rather inhomogeneous over the biological tissue, the accuracy of conventional FLASH imaging methods turns out to be insufficient. In Sedlacik et al., "Calibration of saturation prepared turbo FLASH B1+ maps by actual flip angle imaging at 7T", Proceedings of ISMRM, 2022, p. 2867, an improved method was proposed, consisting in remapping the measured RF magnetic field $B_1$. Despite being an improvement, the resulting accuracy was spatially dependent, since the inaccuracies inherent in the turbo FLASH method are themselves spatially dependent and this is not captured by the linear mapping used in Sedlacik et al.

Finding a compromise between acquisition time and accuracy is a generic problem that arises not only in the determination of magnetic fields in the context of MRI, but also in other medical techniques that deal with the determination of physical quantities on biological tissue.

It is an object of the present invention to provide a computing system for an accurate and time-efficient determination of a physical quantity on a biological tissue, especially soft biological tissues. The present invention is particularly focused on an accurate and time-efficient determination of the radiofrequency magnetic field $B_1$ in the context of MRIs of mammal organs such as the brain.

The purpose of this invention is achieved through a system with the features disclosed in claim 1 and a computer-implemented method with the features detailed in claim 13. Preferred embodiments of the invention with advantageous features are given in the dependent claims.

A first aspect of the invention provides a computing system for providing a mapping of a physical quantity on a biological tissue, comprising: an input data interface, configured to obtain data from the physical quantity on different spatial points of the biological tissue; a computation module, having at least: (a) a digitization unit, configured to produce a digitized representation of the biological tissue in voxels and/or pixels, wherein, based on the obtained data, a subset of the voxels and/or pixels is assigned a value of the physical quantity; (b) a concatenation unit, configured to spatially correlate the voxels and/or pixels of the produced digitized representation of the biological tissue; and (c) a regression unit, configured to process the information of the spatially correlated voxels and/or pixels and generate a regression analysis of the physical quantity on the biological tissue; and an output data interface, configured to, based on the generated regression analysis, provide a mapping of the physical quantity on the biological tissue, wherein a value of the physical quantity is assigned to each voxel and/or pixel.

Biological tissue refers in this invention mostly to soft biological tissue, in particular organs such as the brain, the kidney, the heart or the liver, of mammals and especially humans.

A physical quantity in the context of this invention is to be understood as any physical property that can be quantified by a measurement and expressed as a scalar field (e.g., temperature field) or a vector field (e.g., magnetic field or velocity field).

Herein and in the forthcoming, a mapping is a correspondence between a physical quantity and the spatial points of a biological tissue, such that there is a value of the physical quantity associated with each spatial point. The mapping provided by the invention can also be referred to as a remapping, a correction or a calibration, since it corrects or calibrates the data from the physical quantity obtained via the input data interface.

A digitization of the physical tissue is a mathematical representation or discretization of the physical tissue in terms of pixels (if the digitization is in two dimensions) or in voxels (if the digitization is in three dimensions).

A spatial correlation of the voxels and pixels comprises any procedure or mechanism by which the voxels and pixels are concatenated in a position space with respect to a system of reference, such that one can define in particular relative distances between the different voxels and/or pixels.

A regression analysis refers to any mathematical procedure or algorithm defining an objective function involving the physical quantity at the different spatial points of the biological tissue, which has to be extremized (maximized or minimized).

The computation module and the different units mentioned in this application are broadly understood as entities capable of acquiring, obtaining, receiving or retrieving generic data and/or instructions through a user interface and/or programming code and/or executable programs or any combination thereof. The computation module and the different units are adapted to run programming code and executable programs and to deliver the results for further processing.

The computation module and its comprising units, or parts thereof, may therefore each contain, at least, a central processing unit, CPU, and/or at least one graphics processing unit, GPU, and/or at least one field-programmable gate array, FPGA, and/or at least one application-specific integrated circuit, ASIC and/or any combination of the foregoing. Each of them may further comprise a working memory operatively connected to the at least one CPU and/or a non-transitory memory operatively connected to the at least one CPU and/or the working memory. Each of them may be implemented partially and/or completely in a local apparatus and/or partially and/or completely in a remote system such as by a cloud computing platform.

All of the elements of the computing system may be realized in hardware and/or software, cable-bound and/or wireless, and in any combination thereof. Any of the elements may comprise an interface to an intranet or the Internet, to a cloud computing service, to a remote server and/or the like.

In particular, the computing system of the invention may be implemented partially and/or completely in a local apparatus, e.g. a computer, in a system of computers and/or partially and/or completely in a remote system such as a cloud computing platform.

In systems based on cloud computing technology, a large number of devices is connected to a cloud computing system via the Internet. The devices may be located in a remote facility connected to the cloud computing system. For example, the devices can comprise, or consist of, equipment, sensors, actuators, robots, and/or machinery in an industrial set-up(s).

The devices can be medical devices and equipment in a healthcare unit. The devices can be home appliances or office appliances in a residential/commercial establishment.

The cloud computing system may enable remote configuring, monitoring, controlling, and maintaining connected devices (also commonly known as 'assets'). Also, the cloud computing system may facilitate storing large amounts of data periodically gathered from the devices, analyzing the large amounts of data, and providing insights (e.g., Key Performance Indicators, Outliers) and alerts to operators, field engineers or owners of the devices via a graphical user interface (e.g., of web applications). The insights and alerts may enable controlling and maintaining the devices, leading to efficient and fail-safe operation of the devices. The cloud computing system may also enable modifying parameters associated with the devices and issues control commands via the graphical user interface based on the insights and alerts.

The cloud computing system may comprise a plurality of servers or processors (also known as 'cloud infrastructure'), which are geographically distributed and connected to each other via a network. A dedicated platform (hereinafter referred to as 'cloud computing platform') is installed on the servers/processors for providing above functionality as a service (hereinafter referred to as 'cloud service'). The cloud computing platform may comprise a plurality of software programs executed on one or more servers or processors of the cloud computing system to enable delivery of the requested service to the devices and its users.

One or more application programming interfaces (APIs) are deployed in the cloud computing system to deliver various cloud services to the users.

A second aspect of the present invention provides a computer-implemented method for providing a mapping of a physical quantity on a biological tissue, comprising the following steps: (a) obtaining data from the physical quantity on different spatial points of the biological tissue; (b) producing (S2) a digitized representation of the biological tissue in voxels and/or pixels, wherein a subset of the voxels and/or pixels gets assigned a value of the physical quantity based on the acquired data; (c) spatially correlating the voxels and/or pixels of the produced digitized representation of the biological tissue; (d) generating, based on the information of the spatially correlated voxels and/or pixels, a regression analysis of the physical quantity on the biological tissue; and (e) providing, based on the generated regression analysis, a mapping of the physical quantity on the biological tissue, wherein a value of the physical quantity is assigned to each voxel and/or pixel.

In particular, the method according to the second aspect of the invention may be carried out by the system according to the first aspect of the invention. The features and advantages disclosed herein in connection with the computing device are therefore also disclosed for the method, and vice versa.

According to a third aspect, the invention provides a computer program product comprising executable program code configured to, when executed, perform the method according to the second aspect of the present invention.

According to a fourth aspect, the invention provides a non-transient computer-readable data storage medium comprising executable program code configured to, when executed, perform the method according to the second aspect of the present invention.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

According to a fifth aspect, the invention provides a data stream comprising, or configured to generate, executable program code configured to, when executed, perform the method according to the second aspect of the present invention.

According to a sixth aspect, the invention provides a medical scanning system, comprising a magnetic resonance image scanner, configured to perform magnetic resonance imaging scans, in particular ultrahigh field magnetic resonance imaging scans with parallel radio-frequency imaging, and the computing system, wherein the computing system is configured to obtain data corresponding to the radio-frequency magnetic field from the magnetic resonance image scanner.

The computing system can be implemented (as part of or connected to) an MRI scanner. In this way, the data of the RF magnetic field obtained, for instance, using FLASH imaging, can be quickly processed and images of the mapping (also referred to as remapping, correction or calibration) can be visualized in, e.g., a video display unit. The computing system of the invention can also be connected to computing devices responsible to produce the MRI images, feeding them with the mappings of the $B_1$ magnetic field. Parts of the computing system of the invention can also be implemented in a cloud computing platform.

One of the main ideas underlying the present invention is to provide a computing system which is configured to generate, based on data coming from the evaluation of a physical quantity (e.g. temperature, magnetization) on a biological tissue, a more accurate mapping (or prediction of the spatial distribution) of the physical quantity based on the results of a regression analysis, wherein the regression analysis uses as input the spatial information and correlations between the different points of a digitized representation of the biological tissue. In the particular example of ultrahigh field MRI, the obtained data can be generated using, e.g., saturation-prepared turbo FLASH (satTFL) imaging, from which a more accurate mapping of the $B_1$ magnetic field (i.e., a mapping closer to the actual $B_1$ magnetic field) can be generated with a regression analysis. This regression analysis, thanks to the implemented information on spatial correlations, is especially adapted to map fields with spatial inhomogeneity. This correction or calibration of the satTFL data can be generated in a matter of seconds.

The computing system of the invention comprises a computation module, which is configured to (a) provide a digital representation of the biological tissue, where at least some of the pixels (or voxels) are assigned a value of the physical quantity; (b) spatially correlate the pixels (or voxels); and (c) generate a regression analysis from which a mapping is provided, where all the pixels (or voxels) are assigned a value of the physical quantity.

The device as described above allows for a simple implementation of a computer-implemented method comprising a number of steps. In one step, data from the physical quantity on different spatial points of a biological tissue is obtained. This data can be generated by any process which can measure the physical quantity. In another step, the biological tissue is digitized, where a subset of the ensuing voxels or pixels gets assigned a value of the physical quantity. In a subsequent step, spatial information of the voxels or pixels of the digitized biological tissue is provided, such that the values of the physical quantity are spatially correlated. This information is then used in another step to generate a regression. In a subsequent step, the regression is used to provide a mapping, which associates every point of the biological tissue with a corresponding value of the physical quantity.

One advantage of the present invention is that the provided mapping furnishes a determination of a physical quantity on a biological tissue, which is accurate and can be performed in a time-efficient fashion. As an example, in the context of MRI scans, the determination of the RF magnetic field $B_1$ can be performed accurately with Actual Flip angle Imaging (AFI), but the time required spans over minutes. More time-efficient solutions are provided by Fast Low Angle Shot magnetic resonance imaging (FLASH imaging, e.g. with satTFL), at the expense of a lower accuracy. The present invention can be provided with the fast-generated data from satTFL as input and produce a more accurate mapping (i.e., a calibration or correction of the satTFL data that falls closer to the actual spatial distribution of the $B_1$ field) in a matter of seconds, which makes the invention amenable for its use in the clinical workflow.

A further advantage of the present invention is that, especially due to the implementation of a spatial correlation between the voxels or pixels of the digitized version of the biological tissue, the regression provided by the system of the invention is very robust when applied to the prediction of physical quantities with spatial-dependent profiles. Accordingly, when the present invention is used in these cases, spatial-dependent inaccuracies can be efficiently reduced. Such a situation arises in ultrahigh field MRI, where the RF magnetic field $B_1$ is known to be spatially inhomogeneous.

Advantageous embodiments and further developments follow from the dependent claims as well as from the description of the different preferred embodiments illustrated in the accompanying figures.

According to some embodiments, refinements, or variants of embodiments, the computation module of the invention further comprises a performance assessment unit, which is configured to evaluate the accuracy of the provided mapping based on a ground truth.

The performance assessment unit can be adapted to assess the system performance quantitatively by using different figures of merit or metrics. In particular, the $R^2$ score, defined as

$$R^2 = 1 - \frac{\sum_i(\kappa_i - \hat{\kappa}_i)^2}{\sum_i(\kappa_i - \bar{\kappa})^2}$$

can be determined, where $\hat{k}_i$ is the value of the physical quantity under consideration at voxel or pixel i coming from the generated mapping; $k_i$ is the value of the physical quantity under consideration at voxel or pixel i from the ground truth; and $\bar{k}$ is the value of the physical quantity under consideration from the ground truth averaged over all voxels or pixels.

Alternatively, one can also use the normalized root mean-squared error (NRMSE), defined as

$$NRMSE = \sqrt{\frac{\sum_i(\kappa_i - \hat{\kappa}_i)^2}{\sum_i(\kappa_i)^2}}$$

The performance assessment unit can be used to analyze the results of each regression and further improve the involved algorithms.

According to some embodiments, refinements, or variants of embodiments, the physical quantity is the radiofrequency magnetic field generated during magnetic resonance imaging.

As already mentioned in the foregoing, a particularly suited application of the present invention arises in the context of magnetic resonance imaging (MRI), and in particular in the mapping of the radiofrequency (RF) magnetic field $B_1$ (also referred to as $B_1^+$). In response to this magnetic field the spins of the protons in the biological tissue change their alignment and subsequently generate a signal via induction, allowing them to be imaged. The amount of change of the alignment depends on $B_1$, hence there is often a need to map this field for calibration or correction purposes.

According to some embodiments, refinements, or variants of embodiments, the magnetic resonance imaging comprises ultrahigh field magnetic resonance imaging with parallel transmission imaging radiofrequency.

Ultrahigh field MRI denotes MRI with static magnetic fields at or above 7 Tesla, requiring RF magnetic fields B1 with frequencies at or above 298 MHz. At these frequencies, the spatial inhomogeneities of the $B_1$ along the examined biological tissue are known to be substantial and have to be considered. Parallel transmission or multichannel transmission imaging RF is a technique that is used to accelerate the acquisition of MRI data. This is achieved by an arrangement of coils, each acting as a RF-transmitter and/or a RF-receiver. Together they build a multichannel Tx/Rx array. The $B_1$ field experienced by the biological tissue is the superposition of such transmitted fields. The coils can be arranged so that the properties of the transmitted $B_1$ magnetic field are optimized, in particular by reducing the inhomogeneities of the $B_1$ field on the biological tissue.

According to some embodiments, refinements, or variants of embodiments, the obtained data of the radiofrequency magnetic field is generated following a Fast Low Angle Shot (FLASH) magnetic resonance imaging.

Saturation-prepared Turbo-FLASH magnetic resonance imaging (or satTFL) is a technique to determine the $B_1$ transmission field which combines a high flip angle saturation pulse, followed by a train of low flip angle RF excitations with a low repetition time. The technique allows for a fast determination of the $B_1$ magnetic field (the acquisition time lasts a few seconds) with a satisfactory accuracy, and is already implemented in some scanning machines as part of the pre-scan adjustments. It is therefore a technique that fulfills the requirements of the invention as a source of the data obtained by the input data interface. FLASH imaging is also known by some manufacturers as spoiled gradient echo (SPGR) or contrast-enhanced fast field echo (CE-FFE-T1 or T1-FFE).

According to some embodiments, refinements, or variants of embodiments, the ground truth comprises a mapping of the radiofrequency magnetic field acquired with Actual Flip angle Imaging (AFI).

Actual Flip angle Imaging (AFI) can be currently considered a "gold standard", in terms of accuracy, in the determination of the transmission RF magnetic field $B_1$, and it is therefore an optimal method to use as ground truth for the figures of merit used by the performance assessment unit to evaluate the accuracy of the mapping provided by the system of the invention.

The AFI is very accurate but also has slow acquisition times. In order for the performance assessment unit to use AFI data, it might be preferable to use AFI from a database of recorded past MRIs.

According to some embodiments, refinements, or variants of embodiments, the computation module further comprises a feature generating unit, configured to compute the volume of the biological tissue and/or the spatial coordinates of its center of mass.

The spatial correlation of the voxels and pixels comprises at least the identification of their three-dimensional Cartesian coordinates referred to a spatial system of reference. This local data can be complemented with global data describing the geometry of the biological tissue as a whole. In this respect, two possible parameters are the volume of the biological tissue and its center of mass.

According to some embodiments, refinements, or variants of embodiments, the regression unit further comprises an artificial intelligence entity, which is adapted to implement at least one machine learning regression algorithm, in particular a random forest tree algorithm and/or a gradient boosting algorithm.

Whenever herein an artificial intelligence entity is mentioned, it shall be understood as a computerized entity able to implement different data analysis methods broadly described under the terms artificial intelligence, machine learning, deep learning or computer learning. The artificial intelligence entity can comprise any realization thereof able to perform a regression. Random forest tree (RFT) and gradient boosting (GB) are two examples of well-tested algorithms which are easy to implement, but the invention is not limited to them, and in particular other options, for instance options including neural networks, are also possible.

According to some embodiments, refinements, or variants of embodiments, the machine learning regression algorithm uses as input a feature matrix comprising the spatial components of each voxel and/or pixel and the value of the physical quantity assigned to it.

In preferred implementations of machine learning algorithms, the voxels and/or pixels are provided with a mask or an annotation, consisting of a feature matrix which combines local information corresponding to each voxel (or pixel), i.e. geometrical information comprising the three-dimensional coordinates and an assigned value of the physical quantity. This local information can be combined with additional information, e.g., global information, such as the volume and center of mass of the biological tissue. These last two quantities can be provided by the feature generating unit.

According to some embodiments, refinements, or variants of embodiments, the computing system further comprises an optimization unit, which is configured to use the evaluation of the performance assessment unit to optimize the machine learning regression algorithm.

In these embodiments, data for the optimization can comprised stored data from previous MRIs or data from the artificial intelligence unit of systems connected to other scanning MRI machines. The optimization unit can be adapted to implement different deep learning or reinforcement learning algorithms.

According to some embodiments, refinements, or variants of embodiments, the biological tissue is brain tissue. Until recently, ultrahigh field MRI applications were mostly devoted to brain scans, and it remains the organ where ultrahigh field MRI is best understood. In this context, the present invention can be used to help diagnose, e.g., aneurysms of cerebral vessels, the occurrence of strokes, brain tumors, brain injury after trauma, multiple sclerosis, disorders of the eye, Alzheimer's disease or epilepsy. Applications using functional MRI (fMRI) are also under the scope of the present invention.

According to some embodiments, refinements, or variants of embodiments, the computer-implemented method of the second aspect of the invention further comprises: creating a feature matrix comprising the spatial components of each voxel and/or pixel and the value of the physical quantity assigned to it, wherein the feature matrix is used as input for generating the regression analysis.

In some embodiments, the feature matrix can also contain information about the volume of the biological tissue and/or the spatial coordinates of its center of mass. In the particular case of the mapping of the radiofrequency $B_1$ magnetic field in the context of ultrahigh field MRI, the use of a feature matrix with a combination of local information (voxel positions and their $B_1$ values) with global information (e.g., the volume of the brain and the position of its center of mass) turns out to be advantageous for improving the performance of some regression algorithms.

Although here, in the foregoing and also in the following, some functions are described as being performed by modules or units, it shall be understood that this does not necessarily mean that such modules or units are provided as entities separate from one another. In cases where one or more modules or units are provided as software, the modules or units may be implemented by program code sections or program code snippets, which may be distinct from one another but which may also be interwoven or integrated into one another.

Similarly, in cases where one or more modules or units are provided as hardware, the functions of one or more modules or units may be provided by one and the same hardware component, or the functions of several modules or units may be distributed over several hardware components, which need not necessarily correspond to the modules or units. Thus, any apparatus, system, method and so on which exhibits all of the features and functions ascribed to a specific module or unit shall be understood to comprise, or implement, said module or said unit. In particular, it is a possibility that all modules or units are implemented by program code executed by the computing device, for example a server or a cloud computing platform.

The above embodiments and implementations can be combined with each other as desired, as far as this is reasonable.

Further scope of the applicability of the present method and system will become apparent from the following figures, detailed description and claims. However, it should be understood that the detailed description and specific examples, while indicating preferred embodiments of the invention, are given by way of illustration only, since various changes and modifications within the spirit and scope of the invention will become apparent to those skilled in the art.

Aspects of the present disclosure will be better understood with reference to the following figures.

In the figures:

Fig. 1    is a schematic depiction of a computing system for providing a mapping of a physical quantity on a biological tissue according to an embodiment of the present invention;

Fig. 2    is a block diagram showing an exemplary embodiment of a computer-implemented method for providing a mapping of a physical quantity on a biological tissue according to the present invention;

Fig. 3    is a schematic illustration of a medical scanning system comprising the computing system according to an embodiment of the present invention, and a magnetic resonance imaging scanner;

Fig. 4    shows two plots, where the performance of two regression algorithms generated with a computing system according to an embodiment of the present invention is compared to the performance of a conventional algo-rithm;

Fig. 5    shows two scattering plots, where the performance of regression algorithm according to an embodiment of the present invention is compared with the performance of a conventional algorithm;

Fig. 6    shows two-dimensional projections (sagittal, coronal and transversal) of a brain B1 map acquired via MRI using an embodiment of the computing system of the invention, compared with the ground truth and a state-of-the-art fitting technique;

Fig. 7    is a schematic block diagram illustrating a computer program product according to an embodiment of the third aspect of the present invention; and

Fig. 8    is a schematic block diagram illustrating a non-transitory computer-readable data storage medium according to an embodiment of the fourth aspect of the present invention.

Parts in the different figures that correspond to the same elements have been indicated with the same reference numerals.

The components in the drawings are not necessarily to scale, emphasis being placed instead upon clearly illustrating the principles of the present disclosure. Likewise, certain components can be shown in generalized or schematic form in the interest of clarity and conciseness. In some instances, well-known structures and devices are shown in block diagram form in order to avoid obscuring the concepts of the present invention.

The numeration of the steps in the methods are meant to ease their description. They do not necessarily imply a certain ordering of the steps. In particular, several steps may be performed concurrently.

The detailed description includes specific details for the purpose of providing a thorough understanding of the present invention. However, it will be apparent to those skilled in the art that the present invention may be practised without these specific details.

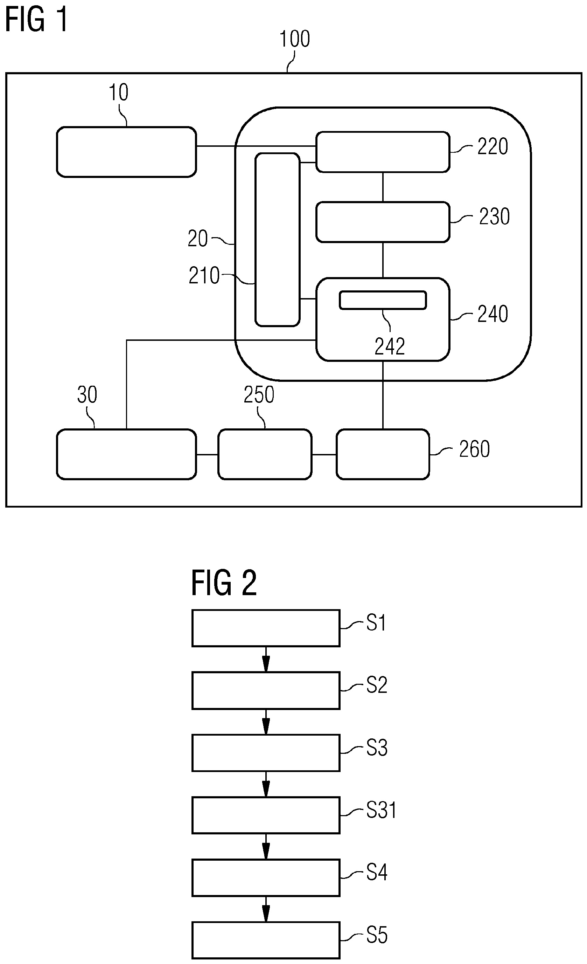

Fig. 1 shows a schematic depiction of a computing system 100 for providing a mapping of a physical quantity on a biological tissue according to an embodiment of the present invention, for instance the mapping of the radio-frequency magnetic field $B_1$ during an ultra-high magnetic resonance imaging with parallel imaging of a brain. In the following, we will describe this particular implementation of the computing system of the invention.

The computing system 100 depicted in Fig. 1 comprises an input data interface 10, a computation module 20, an output data interface 30, a performance assessment unit 250 and an optimization unit 260.

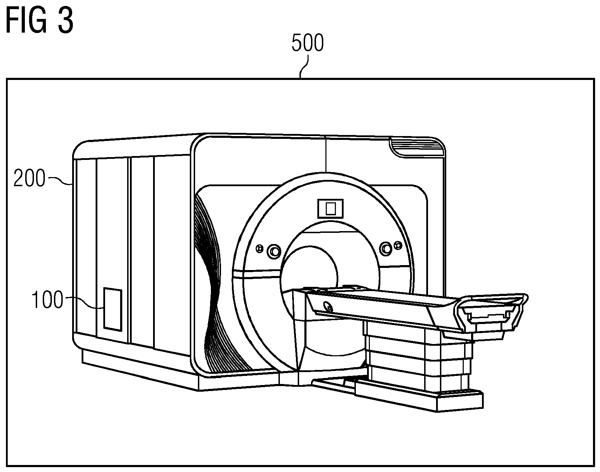

The input data interface 10 is configured to obtain data from the radio-frequency magnetic field $B_1$ on different spatial points of the brain. This data can originate, e.g., from the magnetic resonance imaging scanner 200 depicted in Fig. 3, which can be endowed with a coil arrangement for parallel imaging (not shown in the figure) and a $B_1$ acquisition unit (not shown in the figure), wherein the $B_1$ acquisition unit is configured to measure the $B_1$ magnetic field, e.g., with a Fast Low Angle Shot (FLASH) magnetic resonance imaging, such as a satTFL. The input data interface 10 can connected to the the $B_1$ acquisition unit of the magnetic resonance imaging scanner 200, wired or wireless or with a combination thereof.

The computation module 20 comprises a feature generating unit 210, a digitization unit 220, a concatenation unit 230, and a regression unit 240.

The digitization unit 220 is configured to generate a digitized representation of the brain in voxels, wherein, based on the obtained data, a subset of the voxels and/or pixels are assigned a value of B1 magnetic field, wherein the assigned $B_1$ value is based on the data obtained through the input data interface 10.

The concatenation unit 230 is configured to spatially correlate the voxels of the generated digitized representation of the brain. This can be achieved by computing the three-dimensional coordinates $\mathbf{r_i}=(x_i, y_i, z_i)$ of each voxel i of the digitized representation of the brain with respect to a common system of reference, such that relative distances between the different voxels can be computed.

The regression unit 240 is configured to process the information of the spatially correlated voxels of the digitized representation of the brain and generate a regression analysis of the radio-frequency magnetic field $B_1$ on the brain.

In the implementation shown in Fig. 1, the regression unit 240 is further configured to implement an artificial intelligence entity 242, which is adapted to implement at least one machine learning regression algorithm, in particular a random forest tree algorithm and/or a gradient boosting algorithm.

The regression algorithms can use as input a feature matrix comprising the spatial components $r_i$ of each voxel and/or pixel i and the value of $B_1$ assigned to it, together with global quantities such as the volume of the biological tissue and/or the spatial coordinates of its center of mass. The spatial components of the voxels and the corresponding values of the $B_1$ magnetic field give local information about the brain, which can be combined with global or topological information, such as the brain volume or the brain center of mass. In some embodiments, the computing system 100 comprises a feature generating unit 210, which is configured to compute global quantities such as the brain volume and/or the spatial coordinates of its center of mass. In these embodiments, the feature generating unit 210 uses as input the information from the digitization unit 220. The global quantities computed by the feature generating unit 210 are then fed into the feature matrix to be used by the regression unit 240 as input.

The output data interface 30 is configured to provide a mapping of the $B_1$ magnetic field on the brain based on the regression analysis generated by the regression unit 240. Through the mapping, a value of the $B_1$ magnetic field is assigned to each voxel.

The embodiment illustrated in Fig. 1 further comprises a performance assessment unit 250, which is configured to evaluate the accuracy of the mapping provided by the output data interface 30. This can be done by using different figures of merit, such as a $R^2$ score metric or a normalized root mean-square error (NRMSE), which compare the $B_1$ field on each voxels obtained from the mapping with a ground truth, such as the $B_1$ determination coming an Actual Flip angle Imaging (AFI) .

Some embodiments of the invention, such as the one depicted in Fig. 1, also comprise an optimization unit 260, which is configured to use the evaluation of the performance assessment unit 250 to optimize the machine learning regression algorithm implemented by the regression unit 240.

Fig. 2 is a block diagram showing an exemplary embodiment of a computer-implemented method for providing a mapping of a physical quantity on a biological tissue according to the present invention. The method can be implemented with the computing system 100 described with respect to Fig. 1. In the following, and for concreteness, the method will be applied to the determination of a mapping for the RF magnetic field $B_1$ during a MRI scan. The method comprises a number of steps.

In one step S1, data from the RF magnetic field $B_1$ is obtained on different spatial points of the brain of a patient during an MRI pre-scan. The data can be produced using FLASH imaging, which has short acquisition times and good accuracy.

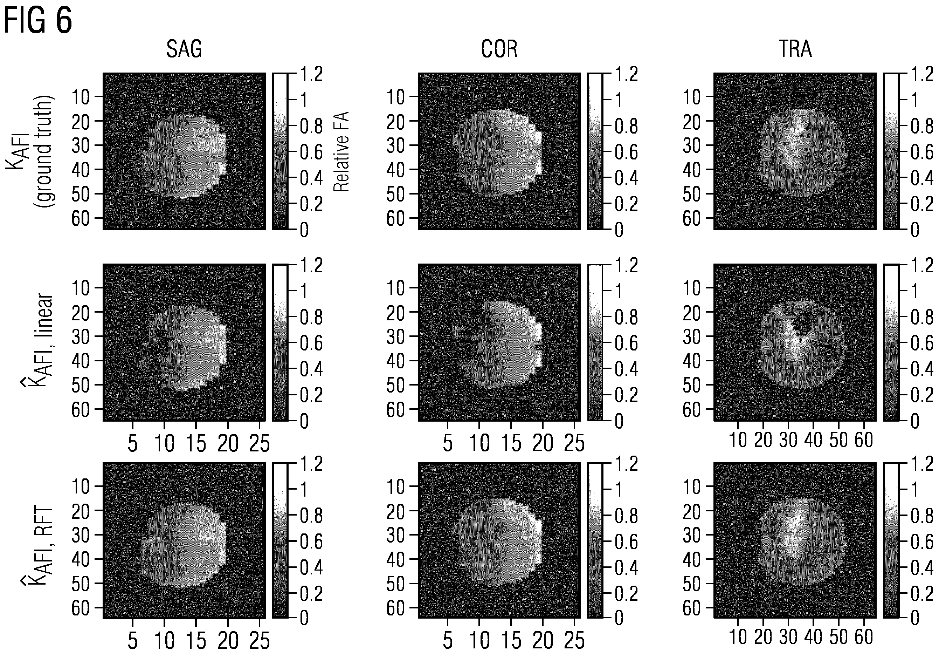

In another step S2, the brain is digitized, where at least a subset of the ensuing voxels gets assigned a value of the $B_1$ field. The subset of voxels that get an assigned value, and the specific assigned value, depend on the characteristics of the data obtained from the FLASH imaging. In some cases, not all the voxels get a value, in some other cases the values of the $B_1$ magnetic fields are interpreted as null because of signal tolerances. In Fig. 6, some of these cases are discussed with respect to illustrative experiments carried by the inventors.

In a subsequent step S3, the voxels of the digitized brain are spatially correlated. This can be done in some cases by assigning spatial coordinates $r_i=(x_i, y_i, z_i)$ to each of the voxels i, with respect to a common spatial frame of reference.

The embodiment depicted in Fig. 2 shows a step S31, in which a feature matrix is created. This feature matrix comprises the spatial coordinates $r_i= (x_i, y_i, z_i)$ of each voxel and the value of the $B_1$ magnetic field assigned to it. In some embodiments, the feature matrix can also contain additional information, e.g., the volume of the brain and/or the spatial coordinates of its center of mass.

This information is then used, in another step S4, to generate a regression analysis. This regression analysis can be implemented with machine learning regression algorithms. In some preferred embodiments, these algorithms comprise a Random Forest Tree (RFT) algorithm and/or a gradient boosting (GB) algorithm. In some other preferred embodiments, the machine learning algorithms may use as input a feature matrix comprising the spatial coordinates $r_i$ of each voxel i, the value of the magnetic field $B_1$ assigned to each voxel i, the volume of the brain and the spatial coordinates of the centre of mass of the brain.

In a subsequent step S5, the generated regression analysis is used to provide a $B_1$ mapping, which associates every point of the brain with a corresponding value of the $B_1$ magnetic field. As a result of the regression, even for voxels where $B_1$ values from the FLASH imaging are unavailable, the mapping can still make a prediction for those voxels using knowledge of their spatial location. The result of the regression is not only a smooth mapping, but one that, with the help of the deployed machine learning algorithms, is closer to the ground truth, i.e. more accurate, than the FLASH imaging and can be provided in a matter of seconds.

Fig. 3 is a schematic illustration of a medical scanning system 500, comprising the computing system according to an embodiment of the present invention, and a magnetic resonance imaging scanner 200.

The magnetic resonance imaging scanner 200 can be a magnetic resonance scanner, preferably configured to perform ultrahigh field magnetic resonance imaging scans with parallel radio-frequency imaging. An example thereof is the MAGNETOM Terra from Siemens Healthcare in Erlangen, Germany.

The computing system 100 is configured to obtain data corresponding to the radio-frequency magnetic field $B_1$ from the magnetic resonance imaging scanner 200. This data is preferably obtained with a Fast Low Angle Shot (FLASH) magnetic resonance imaging, such as satTFL.

In the embodiment illustrated in Fig. 3, the computing system 100 or parts thereof are integrated in the magnetic resonance imaging scanner 200. Parts of the computing system 100 that are not integrated in the magnetic resonance imaging scanner 200 can be connected to it.

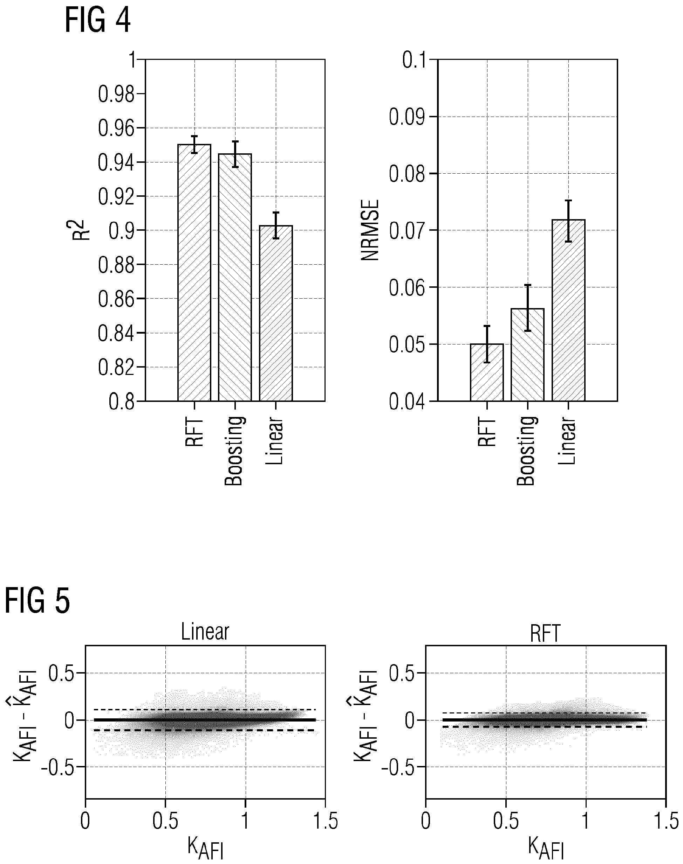

Fig. 4 shows two plots, where the performance of two regression algorithms generated with a computing system according to an embodiment of the present invention is compared to the performance of a conventional algorithm.

The plots of Fig. 4 correspond to a test performed with 26 healthy volunteers, who underwent a scan on the MAGNETOM Terra 7T scanner of Siemens Healthcare in Erlangen, Germany. The parallel imaging was arranged with a 8Tx/32Rx head coil with 8 transmission channels and 32 receiving channels.

Sets of brain B1 maps were acquired for each volunteer using Actual Flip angle Imaging (AFI) and saturation-prepared turbo FLASH (satTFL) imaging, with channels combined in circular polarisation with matched imaging positions, fields of view (FOV) and resolutions. The AFI consisted of a FOV of 256X256X240mm3, a 64X64X48 voxel matrix, nominal flip angle of 60° and a typical scanning time (per channel) of 3min 16s. In turn, the satTFL comprised 25 slices of 5mm thickness with a 5mm spacing, in-plane FOV of $256\times256mm^2$, a 64X64X25 voxel matrix, a nominal flip angle of 90° and a typical scanning time (per channel) of 8s.

Each of the scanned brains were digitized. A feature matrix was used for each scan, comprising the spatial positions of each voxel, the magnitude value of the $B_1$ field on each voxel, the volume of the brain and the position of its center of mass. This 8-dimensional feature matrix was fed into a Random Forest Tree (RFT) algorithm and a Gradient Boosting (GB) algorithm. The algorithms were trained using the AFI results as ground truth. The training consisted of 26 folds, where for each fold one of the 26 datasets was excluded from the training and left as a test dataset. The left-out dataset was predicted after each training fold and compared to the ground truth (the AFI value). The algorithm performance was assessed quantitatively using as figures of merit an $R^2$ and a NRMSE metric.

Fig. 4 shows the results obtained for the $R^2$ (left panel) and the NRMSE (right panel) metrics for the RFT and the GB algorithms, averaged over the 26 folds.

The RFT algorithm was trained with a training configuration of 362 trees, 242460 maximum tree branch splits, a minimum of 5 samples per leaf node and 3 features sampled at each tree split. The GB algorithm was implemented with a least squares loss function and trained with a training configuration of 362 trees, 242460 maximum tree branch splits, a minimum of 5 samples per leaf node and a learning rate of 0.1.

In Fig. 4, the results obtained for the RFT and GB algorithms are compared with the linear fitting method of Sedlacik et al., "Calibration of saturation prepared turbo FLASH B1+ maps by actual flip angle imaging at 7T", Proceedings of ISMRM, 2022, p. 2867, noted as "linear" in the plots. Sedlacik et al. use only the values of the B1 magnetic field as feature matrix, while the computing system of the invention additionally takes into account geometric features, both local (e.g., the spatial coordinates of the voxels) and global (e.g., the volume of the brain and/or the coordinates of its center of mass) and concatenates them.

The plots in Fig. 4 show a superior performance of the system of the invention with respect to the prior state of the art, with a slightly better performance obtained with RFT.

An overfitting study of the regression algorithms was also performed by comparing the $R^2$ score shown in Fig. 4 with the values obtained when test datasets to be predicted belonged to the training set. The average $R^2$ for RFT moved from 0.951 to 0.977, while for GB the average $R^2$ moved from 0.945 to 0.998. The smaller increase for RFT indicates that RFT is more robust against overfitting.

The algorithms of the computing system of the invention performed with an average accuracy of 0.95 when compared with the AFI data (the ground truth) and provided the mapping (the correction or calibration of the satTFL data) for each volunteer in a matter of 2 seconds, which is quick enough to be used in clinical workflows.

Fig. 5 shows two scattering plots, where the performance of regression algorithm according to an embodiment of the present invention is compared with the performance of a conventional algorithm. The results in Fig. 5 correspond to the same case study already detailed with respect to Fig. 4. The (scattered) plots of Fig. 5 show the difference between $\hat{k}_{AFI}$ and $\hat{k}_{AFI}$, i.e., the difference between the $B_1$ magnetic field and the ground truth (obtained with AFI) for each of the voxels of all the tested brains, normalized to the nominal flip angle. The results of the linear method of Sedlacik et al. (left panel) show a larger spread compared to the results obtained with the RFT method.

Fig. 6 shows two-dimensional projections (SAG standing for sagittal, COR standing for coronal, and TRA standing for transversal) of a brain B1 map acquired via MRI using an embodiment of the computing system of the invention and comparing it with the ground truth and a state-of-the-art fitting technique. The setting are the same detailed in Fig. 4. Fig. 6 shows contour plots of the $B_1$ magnetic field and the relative flip angle (relative FA) of one of the brain MRI, where the first row shows the results for the ground truth (obtained with AFI), the second row the results of the linear method of Sedlacik et al. and the third row the results of using a RFT regression algorithm with the computing system of the invention.

Fig. 6 shows that the RFT regression algorithm is more accurate than the linear fitting, and in particular it can "fill in" voxels that are missing in the acquired data from satTFL. Moreover, the RFT regression algorithm is clearly superior when it comes to map the spatial inhomogeneity of the $B_1$ magnetic field, even for very small values of the flip angle (FA). This is a manifestation of the capacity of the RFT regression algorithm to leverage the geometric features introduced therein (in particular the spatial correlation of the voxels) for a more accurate mapping of the $B_1$ magnetic field.

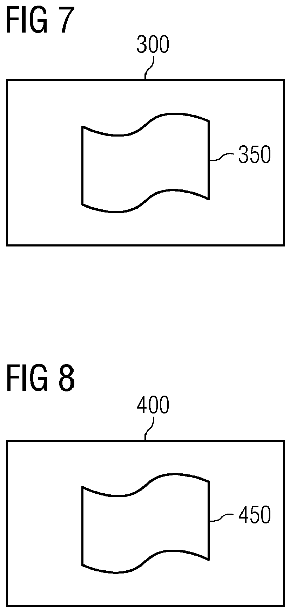

Fig. 7 shows a schematic block diagram illustrating a computer program product 300 according to an embodiment of the third aspect of the present invention. The computer program product 300 comprises executable program code 350 configured to, when executed, perform the method according to any embodiment of the second aspect of the present invention, in particular as it has been described with respect to the preceding figures.

Fig. 8 shows a schematic block diagram illustrating a non-transitory computer-readable data storage medium 400 according to an embodiment of the fourth aspect of the present invention. The data storage medium 400 comprises executable program code 450 configured to, when executed, perform the method according to any embodiment of the second aspect of the present invention, in particular as it has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure. Thus, the present invention is not intended to be limited to the embodiments shown herein but it is to be accorded the widest scope consistent with the principles and novel features disclosed herein. Therefore, the present invention is not to be limited except in accordance with the following claims.

**Claims**

1. A computing system (100) for providing a mapping of a physical quantity on a biological tissue, comprising:

   an input data interface (10), configured to obtain data from the physical quantity on different spatial points of the biological tissue;
   a computation module (20), having at least:

   a digitization unit (220), configured to produce a digitized representation of the biological tissue in voxels and/or pixels, wherein, based on the obtained data, a subset of the voxels and/or pixels are assigned a value of the physical quantity;
   a concatenation unit (230), configured to spatially correlate the voxels and/or pixels of the produced digitized representation of the biological tissue; and
   a regression unit (240), configured to process the information of the spatially correlated voxels and/or pixels and generate a regression analysis of the physical quantity on the biological tissue; and
   an output data interface (30), configured to, based on the generated regression analysis, provide a mapping of the physical quantity on the biological tissue, wherein a value of the physical quantity is assigned to each voxel and/or pixel.

2. The computing system (100) according to claim 1, wherein the computation module (20) further comprises a performance assessment unit (250), which is configured to evaluate the accuracy of the provided mapping based on a ground truth.

3. The computing system (100) according to any of the previous claims, wherein the physical quantity is the radiofrequency magnetic field generated during magnetic resonance imaging, MRI.

4. The computing system (100) according to claim 3, wherein the magnetic resonance imaging, MRI, comprises ultrahigh field magnetic resonance imaging with parallel transmission imaging radiofrequency.

5. The computing system (100) according to claim 4, wherein the obtained data of the radiofrequency magnetic field is generated following a fast low angle shot magnetic resonance imaging.

6. The computing system (100) according to claim 2 and any of the claims 3 to 5, wherein the ground truth comprises a mapping of the radiofrequency magnetic field acquired with actual flip angle imaging.

7. The computing system (100) according to any of the previous claims, wherein the computation module (20) further comprises a feature generating unit (210), configured to compute the volume of the biological tissue and/or the spatial coordinates of its center of mass.

8. The computing system (100) according to any of the previous claims, wherein the regression unit (240) further comprises an artificial intelligence entity (242), which is adapted to implement at least one machine learning regression algorithm, in particular a random forest tree algorithm and/or a gradient boosting algorithm.

9. The computing system (100) according to claim 8, wherein the machine learning regression algorithm uses as input a feature matrix comprising the spatial components of each voxel and/or pixel and the value of the physical quantity assigned to it.

10. The computing system (100) according to claim 2 and any of the claims 8 or 9, further comprising an optimization unit (260), which is configured to use the evaluation of the performance assessment unit (250) to optimize the machine learning regression algorithm.

11. The computing system (100) according to any of the previous claims, wherein the biological tissue is brain tissue.

12. A medical scanning system (500), comprising a magnetic resonance imaging scanner (200), configured to perform magnetic resonance imaging scans, in particular ultrahigh field magnetic resonance imaging scans with parallel radio-frequency imaging, and the computing system (100) according to any of claims 3 to 6, wherein the computing system (100) is configured to obtain data corresponding to the radio-frequency magnetic field from the magnetic resonance imaging scanner (200).

13. A computer-implemented method for providing a mapping of a physical quantity on a biological tissue, preferably to be implemented with the computing system (100) according to any of the claims 1 to 12, comprising the following steps:

    obtaining (S1) data from the physical quantity on different spatial points of the biological tissue;
    producing (S2) a digitized representation of the biological tissue in voxels and/or pixels, wherein a subset of the voxels and/or pixels gets assigned a value of the physical quantity based on the acquired data;

    spatially correlating (S3) the voxels and/or pixels of the produced digitized representation of the biological tissue;
    generating (S4), based on the information of the spatially correlated voxels and/or pixels, a regression analysis of the physical quantity on the biological tissue; and
    providing (S5), based on the generated regression analysis, a mapping of the physical quantity on the biological tissue, wherein a value of the physical quantity is assigned to each voxel and/or pixel.

14. The computer-implemented method according to claim 13, further comprising: creating (S31) a feature matrix comprising the spatial components of each voxel and/or pixel and the value of the physical quantity assigned to it, wherein the feature matrix is used as input for generating (S4) the regression analysis.

15. A computer program product (300) comprising executable program code (350), which is configured, when executed, to perform the computer-implemented method according to claim 13 or claim 14.

16. A non-transient computer-readable data storage medium (400) comprising executable program code (450), which is configured, when executed, to perform the computer-implemented method according to claim 13 or claim 14.

FIG 1

FIG 2

FIG 3

FIG 4

FIG 5

# FIG 6

EP 4 431 968 A1

FIG 7

300

350

FIG 8

400

450

Europäisches
Patentamt

European
Patent Office

Office européen
des brevets

# EUROPEAN SEARCH REPORT

Application Number

EP 23 16 2592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAN SEDLACIK ET AL: "Calibration of saturation prepared turbo FLASH B1+ maps by actual flip angle imaging at 7T", PROCEEDINGS OF THE JOINT ANNUAL MEETING ISMRM-ESMRMB 2022 & ISMRT ANNUAL MEETING, LONDON, UK, 07-12 MAY 2022, ISMRM, 2030 ADDISON STREET, 7TH FLOOR, BERKELEY, CA 94704 USA, no. 2867, 22 April 2022 (2022-04-22), XP040729415, * the whole document * | 1-16 | INV. G01R33/56 G01R33/24 G01R33/561 G06N3/02 |
| X | US 2021/018583 A1 (GUI DAWEI [US] ET AL) 21 January 2021 (2021-01-21) <br><br> * figures 1,2 * <br> * paragraph [0112] - paragraph [0122] * | 1-3, 7-10, 12-16 | |
| X | LUDWIG ET AL.: "Correction of B1-field inhomogeneity of surface coils in the analysis of DCE-MRI experiments", PROCEEDINGS OF THE INTERNATIONAL SOCIETY FOR MAGNETIC RESONANCE IN MEDICINE, ISMRM, 12TH SCIENTIFIC MEETING AND EXHIBITION, KYOTO, JAPAN, 15-21 MAY 2004, 1 May 2004 (2004-05-01), XP040592104, * the whole document * | 1-3,7,9, 12-16 | **TECHNICAL FIELDS SEARCHED (IPC)** <br><br> G01R <br> G06N |
| T | DUBINER MICHAEL ET AL: "A Machine-Learning Approach for Saturation-Prepared Turbo FLASH B1+ Maps Calibration", MACHINE LEARNING, vol. 45, no. 1, 19 May 2023 (2023-05-19), pages 5-32, XP093072351, New York ISSN: 0885-6125, DOI: 10.1023/A:1010933404324 * the whole document * | | |

The present search report has been drawn up for all claims

| Place of search | Date of completion of the search | Examiner |
|---|---|---|
| Munich | 18 August 2023 | Durst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 2592

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2023

| Patent document cited in search report | | Publication date | Patent family member(s) | | Publication date |
|---|---|---|---|---|---|
| US 2021018583 | A1 | 21-01-2021 | CN | 112240995 A | 19-01-2021 |
| | | | US | 2021018583 A1 | 21-01-2021 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SEDLACIK et al.** Calibration of saturation prepared turbo FLASH B1+ maps by actual flip angle imaging at 7T. *Proceedings of ISMRM,* 2022, 2867